**Europäisches Patentamt**

**European Patent Office**

**Office européen des brevets**

(19)

(11) Veröffentlichungsnummer: **0 107 782**

**A1**

(12) # EUROPÄISCHE PATENTANMELDUNG

(21) Anmeldenummer: 83109411.5

(22) Anmeldetag: 21.09.83

(51) Int. Cl.³: **C 07 G 15/00**
C 07 C 103/52, A 23 J 1/00
A 61 K 35/00, A 61 K 37/24

(30) Priorität: 30.09.82 DE 3236267

(43) Veröffentlichungstag der Anmeldung:
09.05.84 Patentblatt 84/19

(84) Benannte Vertragsstaaten:
AT BE CH DE FR GB IT LI LU NL SE

(71) Anmelder: Serono Pharmazeutische Präparate GmbH
Merzhauser Strasse 134
D-7800 Freiburg(DE)

(72) Erfinder: Voelter, Wolfgang, Prof. Dr.
Panoramastrasse 71
D-7400 Tübingen-Hagelloch(DE)

(72) Erfinder: Lippert, Theodor, Prof. Dr.
Erlenweg 38
D-7400 Tübingen(DE)

(72) Erfinder: Entenmann, Astrid Heidrun
Grüningerstrasse 6
D-7000 Stuttgart 70(DE)

(72) Erfinder: Burgardt, Arno
Hugo-Junkerstrasse 13
D-8022 Gründwald(DE)

(74) Vertreter: Kraus, Walter, Dr. et al,
Patentanwälte Kraus & Weisert Irmgardstrasse 15
D-8000 München 71(DE)

(54) Verfahren zur Herstellung von Relaxin.

(57) Die Erfindung betrifft ein Verfahren zur Herstellung von Relaxin sowie ein Verfahren zur Hochreinigung von Relaxin, bei dem als Ausgangsmaterial Spermienflüssigkeit, Mucosa, Teile und Ausscheidungsprodukte des Magenund Darmtrakts, Ovarien oder Corpus luteum verwendet wird.

Die Erfindung betrifft weiterhin das bei dem Verfahren erhaltene Relaxin und ein Arzneimittel, welches das Relaxin, welches man bei dem Verfahren erhält, enthält.

EP 0 107 782 A1

SERONO PHARMAZEUTISCHE PRÄPARATE GMBH
7800 Freiburg


Verfahren zur Herstellung von Relaxin


Die Erfindung betrifft ein Verfahren zur Herstellung von Relaxin sowie ein Verfahren zur Hochreinigung von Relaxin, das bei dem Verfahren erhaltene Relaxin und ein Arzneimittel, welches das bei dem Verfahren erhaltene Relaxin enthält.

Relaxin wurde erstmals 1926 von Frederick Hisaw als Hormon beschrieben. Er entdeckte den Wirkstoff, als er aus trächtigen Kaninchen gewonnenes Blutserum Meerschweinchen injizierte und eine Relaxation des Symphysenligaments feststellte.

In den folgenden Jahren haben sich kaum irgendwelche Arbeitskreise mit der Darstellung von Relaxin befaßt. Erst mit Beginn der siebziger Jahre gelang es Sherwood und O'Byrne, sehr hoch gereinigtes Relaxin durch säulenchromatographische Methoden zu erhalten. Die Reinigungsverfahren von Sherwood et al. (O.D. Sherwood und E.M. O'Byrne: Arch. Biochem. Biophys. 160, 185 (1974)) sind bis heute die am häufigsten und wirkungsvollsten Methoden zur Gewinnung von hochreinem Relaxin.

Später wurde Relaxin nicht nur in den Organen des Genitaltrakts, sondern auch im Blutserum verschiedener Säugetierespezies (Homo sapiens, Rind, Schwein, Hund, Ratte und Maus) meist während der Schwangerschaft nachgewiesen. Hauptpro-

- 2 -

0107782

duktionsort des Hormons ist wahrscheinlich das corpus luteum des Ovars. Relaxin wird dort zusammen mit Östrogen, Progesteron und Androgen produziert. Für die Isolierung wurden hauptsächlich die Ovarien trächtiger Schweine verwendet, weil hier das Relaxin in beträchtlich höherer Konzentration als bei anderen Säugetieren vorkommt.

Schweine- und Hairelaxin sind bis heute, neben Rattenrelaxin, das einzige Hormon dieser Art, dessen Primärstruktur durch Sequenzierung des isolierten Peptids bekannt ist. Allerdings wurde Relaxin nicht nur bei weiblichen sondern auch bei männlichen Säugetieren (Mensch) wie auch bei männlichen (Huhn) und weiblichen Nichtsäugern (Hai) gefunden.

Bei Mensch und Huhn fand man das Relaxin in den Testes. Bemerkenswert ist, daß Relaxin aber nicht im Hoden oder Blutserum von Bären oder Ratten nachweisbar ist. Relaxin erhöht bei männlichen Spezies die Spermienimmobilität.

Die Konzentration des Relaxins im Blutserum erhöht sich im Laufe der Trächtigkeit bei den Tieren stark. Häufig findet man einen Konzentrationsanstieg kurz vor der Geburt.

Man nimmt an, daß das corpus luteum die häuptsächliche Relaxinquelle von Frauen ist, insbesondere während der Schwangerschaft, wo es im Blut nachweisbar ist. Bei nichtschwangeren Frauen ist die Bildung von Relaxin so gering, daß der Nachweis im Blut kaum möglich ist. Kürzlich wurde das Hormon, von dem man annahm, daß es nur bei Frauen vorkommt, auch in humanem Samenplasma nachgewiesen.

In der folgenden Tabelle ist der Gehalt an relaxinhaltiger Substanz in humaner Milch und im Blutplasma zu unterschiedlichen Zeitpunkten nach der Geburt angegeben.

Tabelle

| Zahl der Patienten | Milch pg/ml (Bereich) | Plasma pg/ml (Bereich) | Zeit nach der Geburt |
|---|---|---|---|
| 9 | 193 (n.n. -700) | 82 (n.n. -203) | Tag 3 |
| 11 | 286 (n.n. -617) | 28 (n.n. -143) | Tag 4 |
| 11 | 391 (n.n. -790) | 19 (n.n. -150) | Tag 5 |
| 7 | 438 (n.n. -810) | 32 (n.n. -223) | Tag 6 |
| 6 | 568 (353 -930) | n.n. | 2-4 Wochen |
| 8 | 464 (177 -741) | n.n. | 5-12 Wochen |
| 4 | 495 (229 -937) | n.n. | 18-34 Wochen |

n.n. = nicht nachweisbar

Es ist bekannt, daß Relaxin vom corpus luteum bei der Schwangerschaft vieler Säugetiere produziert wird. Es war lange Zeit nicht bekannt, ob die Plazenta eine weitere Rohstoffquelle zur Herstellung von Humanrelaxin ist (1. M. X. Zarrow, E. G. Holmstrom und H. A. Salhanick: J. Clin. Endocr. 15, 22 (1955); F. D. Dallenbach und G. Dallenbach-Hellweg: Virchows Arch. Path. Anat. Physiol. 337, 301 (1964) und G. Weiss, E. M. O'Byrne, B.C. Steinetz: Science 194, 948 (1970)). Es scheint nun, daß die menschliche Dezidua Relaxin enthält (M. Bigazzi, F. Nardi, P. Bruni und G. Petrucci: J. Clin. Endocrinol. Metab. 51, 939 (1980); S. Yamamoto, S. C. M. Kwok, F. C. Greenwood und G. D. Bryant-Greenwood: J. Clin. Endocrinol. Metab. 52, 601 (1981); S. Krassnigg, H. K. Rjosk, G. K. Stalla und K. von Werden: Acta Endocrinol. Suppl. 246, 99 (1982)).

01077782

Neuere Untersuchungen haben gezeigt, daß dem , insbesondere dem menschlichen, Relaxin physiologische Wirkungen zukommen und daß es heute von großem klinischen Interesse ist.

Relaxin zeigt Wirkungen auf den Uterus, beispielsweise beeinflußt es die Uterusmotilität und bewirkt eine Erweichung der Cervix. In-vitro- und In-vivo-Untersuchungen an Ratten und Mäuseuteri zeigten eine Verhinderung spontaner Kontraktionen des Myometriums durch Relaxin. Viele biochemische Veränderungen, die in der Cervix stattfinden, werden durch Relaxin induziert. Es handelt sich hierbei um die Aufnahme von Wasser, Glycogen, die Depolymerisation der Grundsubstanz und die Aktivierung der Kollagenpeptidase.

Relaxin zeigt weiterhin Wirkungen auf das Vaginalephithel, indem es verhornend wirkt, und Wirkung auf das Wachstum der Brustdrüsen. Relaxin wirkt mit Progesteron und Östrogen synergistisch und ergibt eine Zunahme des lobuloalveolären Gewebes. Es bewirkt weiterhin eine Erweiterung des Symphysenligaments.

Man unterscheidet Schweine-, Rinder-, Ratten- etc. -relaxine, je nachdem, woraus die betreffende Substanz gewonnen wurde. Die verschiedenen Relaxine sind alle Polypeptidhormone, die sich teilweise geringfügig in ihrer Aminosäuresequenz, im Molekulargewicht und in ihrer Relaxinaktivität unterscheiden.

Gemeinsam sind dem Relaxin jedoch zwei Peptidketten, welche durch zwei Cystinreste verknüpft sind. Außerdem zeigen die Relaxine alle ähnliche pharmakologische Wirkungen.

In der US-PS 2 852 431 wird ein Verfahren zur Extraktion und Reinigung von Relaxis aus Ovarien trächtiger Tiere beschrieben. In der DE-OS 3 102 487 wird ein Verfahren zur Herstellung von Humanrelaxin aus Fötusmembranen beschrieben. Diese bekannten Verfahren besitzen jedoch den Nachteil, daß Ausgangsmaterialien verwendet werden, die schwer erhältlich sind und nur in geringem Ausmaß zur Verfügung stehen. Außerdem umfaßt das Verfahren zur Isolierung und Reinigung zahlreiche Stufen und ist somit arbeitsaufwendig und sehr kostspielig. In der Literaturstelle: Hansjürgen Struck "Untersuchungen über Reinigung, Bestimmung und Wirkung des Relaxins" in Forschungsberichte des Landes Nordrheinwestfalen, Nr. 2304 wird über die Reinigung des Relaxins, seine Bestimmung und seine Wirkung berichtet.

Nähere Einzelheiten hinsichtlich der Reinigung und des Nachweises von Relaxin können einer kürzlich erschienenen Zusammenfassung (Gillian D. Bryant-Greenwood, Endocrine Reviews, Bd. 3, Nr. 1, S. 62, 1982) und den dort genannten Literaturstellen entnommen werden. Auf diese Literaturstelle und die dort genannten Zitate wird expressis verbis Bezug genommen.

M. J. Fields et al berichten kürzlich (Annals of the New York Academy of Sciences, Vol. 380, Seite 36 ff (1982)) über ein neues Reinigungsverfahren von Relaxin, bei dem diese Autoren u. a. eine Gelpermeation und Ionenaustauschchromatographie durchführen.

Bis heute gibt es jedoch keine Möglichkeit, Relaxin in größeren Mengen und auf einfache Weise zu gewinnen. Wie oben ausgeführt wurde, stehen die Ausgangsmaterialien zur Isolierung von Relaxin nur in geringen Mengen zur Verfügung und daher ist Relaxin nur in äußerst beschränktem Umfang zugänglich. Die Totalsynthese von Relaxin konnte noch nicht durchgeführt werden, und es ist anzunehmen, daß Relaxin, das durch Totalsynthese hergestellt wird, auch sehr teuer ist. Nachdem dem Relaxin physiologische Wirkungen zu-

0107782

zuschreiben sind, besteht ein Bedarf an einem Verfahren, gemäß dem Relaxin auf einfache Weise erhalten werden kann.

Bei den bekannten Verfahren wird außerdem ein Relaxin erhalten, dessen pharmakologische Wirksamkeit von Herstellungsverfahren zu Herstellungsverfahren schwankt und weiterhin von dem Material abhängt, aus dem das Relaxin gewonnen wird. Dies legt die Vermutung nahe, daß das gemäß den bekannten Verfahren dargestellte und erhaltene Relaxin nicht einheitlich ist und möglicherweise noch Verunreinigungen enthält, die sich in der Vergangenheit nicht haben abtrennen lassen.

Der vorliegenden Erfindung liegt daher die Aufgabe zugrunde, ein Verfahren zur Verfügung zu stellen, gemäß dem Relaxin auf einfache Weise aus billigen, leicht zugänglichen Ausgangsstoffen isoliert und erhalten werden kann.

Erfindungsgemäß soll weiterhin ein Relaxin zur Verfügung gestellt werden, das konstante Eigenschaften aufweist, insbesondere bei den verwendeten Nachweistests immer gleiche Reaktionen zeigt und welches auch gleiche pharmakologische Wirkungen zeigt. Erfindungsgemäß soll ein Relaxin zur Verfügung gestellt werden, dessen Eigenschaften nicht von seinem Herstellungsverfahren und von den zu seiner Herstellung verwendeten Ausgangsmaterialien abhängen. Erfindungsgemäß soll ein Verfahren zur Verfügung gestellt werden, mit dem hochreines Relaxin erhalten werden kann.

0107782

Die Erfindung betrifft somit ein Verfahren zur Herstellung von Relaxin, das dadurch gekennzeichnet ist, daß man ein gegebenenfalls vorbehandeltes relaxinhaltiges Ausgangsmaterial

(1)     mit einer starken Säure bis zu einem pH-Wert von 0,1 bis 2,5 bei einer Temperatur im Bereich von der Gefriertemperatur bis 15°C im Verlauf von 5 Minuten bis 30 Minuten ansäuert,

(2)     das Reaktionsgemisch während einer Zeit von 30 Minuten bis 24 Stunden bei einer Temperatur im Bereich von der Gefriertemperatur bis 15°C stehen läßt,

(3)     den Rückstand bei einer Temperatur im Bereich von der Gefriertemperatur bis 15°C abtrennt,

(4)     zu der erhaltenen klaren Flüssigkeit bei einer Temperatur im Bereich von der Gefriertemperatur bis 15°C   50 bis 200 Vol-%, bezogen auf das Volumen der Flüssigkeit, an einem in Wasser löslichen, organischen Lösungsmittel zugibt,

(5)     die Stufe 2 wiederholt,

(6)     die Stufe 3 wiederholt,

(7)     zu der erhaltenen Flüssigkeit das 1- bis 10fache Volumen eines auf eine Temperatur von -10°C bis -70°C gekühlten, in Wasser löslichen, organischen Lösungsmittels zugibt, wobei dieses Lösungsmittel das gleiche oder ein anderes wie bei der Stufe 4 sein kann,

(8)     die Stufe 2 gegebenenfalls wiederholt,

(9)  den das Relaxin enthaltenden Niederschlag bei
     einer Temperatur im Bereich von der Gefriertemperatur bis 15°C abtrennt,

(10) den Niederschlag zu der 0,5- bis 5fachen
     Volumenmenge einer Pufferlösung zugibt und
     gegebenenfalls filtriert,

(11) gegebenenfalls 0,1 bis 0,5% Natriumazid und gegebenenfalls 0,05 bis 5 mM Phenylmethansulfonylfluorid oder
     -chlorid zu dem erhaltenen Reaktionsgemisch zugibt,

(12) das Reaktionsgemisch bzw. die das Relaxin enthaltenden Fraktionen über ein Ultramembranfilter
     filtriert, welches eine Ausschlußgrenze von
     10 000 Dalton aufweist, und/oder

(13) das Reaktionsgemisch bzw. die das Relaxin
     enthaltenden Fraktionen einer Ionenaustauschchromatographie unterwirft,

(14) das Reaktionsgemisch bzw. die das Relaxin ent-
     haltenden Fraktionen einer Gelfiltration unter-
     wirft,

     wobei die Stufen 12, 13 und 14 in beliebiger
     Reihenfolge und mehrmals durchgeführt werden
     können und wobei die Stufe 14 zwingend ist und
     mindestens eine der Stufen 12 oder 13 durchgeführt werden muß und

(15) die das Relaxin enthaltenden Fraktionen einengt
     und das gereinigte Relaxin gewinnt.

Die Erfindung betrifft weiterhin ein Verfahren zur Hochreinigung von Relaxin, das dadurch gekennzeichnet ist, daß man eine Lösung von gereinigtem Relaxin einer Hochreinigungsstufe unterwirft, indem man eine

(16) Umkehrphasenchromatographie oder/und

(17) eine HPLC-Ionenaustauschchromatographie oder/und

(18) HPLC-Gelchromatographie oder/und

(19) ein Batch-Verfahren mittels Ionenaustauschchromatographie mit anschließender Elution und

(20) gegebenenfalls eine Gelchromatographie durchführt und

(21) die das Relaxin enthaltende Fraktion einengt und das hochgereinigte Relaxin gewinnt, wobei die Stufen 16 bis 19 gegebenenfalls beliebig wiederholt werden können und wobei mindestens zwei der Stufen 16 - 19 durchgeführt werden müssen.

Die Erfindung betrifft außerdem das bei dem Verfahren erhaltene Relaxin. Sie betrifft außerdem ein Arzneimittel, das dadurch gekennzeichnet ist, daß es neben üblichen Trägerstoffen und Verdünnungsmitteln Relaxin, das bei dem obigen Verfahren erhalten worden ist, und insbesondere Relaxin, welches aus Spermienflüssigkeit, Mucosa, Teilen und Ausscheidungsprodukten des Magen- und Darmtrakts, Ovarien oder Corpus luteum erhalten worden ist, enthält.

Bei dem erfindungsgemäßen Verfahren verwendet man als Ausgangsmaterial ein relaxinhaltiges Material, wie Spermienflüssigkeit, Mucosa, Teile und Ausscheidungsprodukte des Magen- und Darmtrakts, Ovarien oder Corpus
luteum. Diese Materialien können von Menschen und Tieren,
wie von Fischen und Säugetieren, stammen. Im allgemeinen
bezieht man die erforderlichen Rohmaterialien aus Kliniken
oder aus Schlachthöfen.

Gegebenenfalls empfiehlt es sich, das Rohmaterial, bevor
es bei dem erfindungsgemäßen Verfahren eingesetzt wird,
vorzubehandeln, um irgendwelche Nebenprodukte abzutrennen.

Verwendet man als Ausgangsmaterial Spermienflüssigkeit,
so behandelt man das Samenejakulat mit 0,01 bis 0,1%
Natriumazid, gibt gegebenenfalls noch 0,05 bis 5 mM Phenylmethansulfonylfluorid oder -chlorid hinzu, trennt das
erhaltene Reaktionsgemisch in Spermatozoa und Spermienflüssigkeit. In der Spermienflüssigkeit ist das Relaxin
enthalten, welches dann für das Verfahren weiterverwendet
wird.

Verwendet man als Ausgangsmaterial frische Plazenta, so
wird diese in eiskalter Ampuwa-Lösung, die 0,01 bis 0,1%
Natriumazid und 0,05 bis 5 mM Phenylmethansulfonylfluorid
oder -chlorid enthält, homogenisiert. Das homogenisierte
Material wird dann bei dem erfindungsgemäßen Verfahren
eingesetzt.

Wird Harn als Ausgangsmaterial verwendet, wird der Harn
im allgemeinen vorbehandelt, um die Lösung einzuengen.
Dazu wird der Harn auf einen pH-Wert im Bereich von 0,1
bis 5, vorzugsweise 0,1 bis 2 angesäuert. Zum Ansäuern
kann man konzentrierte oder verdünnte Salzsäure, Schwefelsäure, Perchlorsäure, Trifluoressigsäure oder Trichloressigsäure verwenden. Diese Säuren können in einer beliebigen Konzentration verwendet werden, wobei die Konzentration jedoch ausreichend hoch sein muß, um den gewünschten pH-Wert zu ergeben. Von dem erhaltenen Reaktionsprodukt wird dann der unlösliche Niederschlag beispielsweise durch Filtration, Zentrifugieren abgetrennt. Die erhaltene Lösung wird gegebenenfalls bei einer Temperatur bis 40°C, bevorzugt bis 20°C, eingeengt. Die Einengung erfolgt, um eine Konzentrierung der Lösung zu erreichen. Sie kann allgemein innerhalb eines Temperaturbereichs von der Gefriertemperatur bis 40°C erfolgen. Besonders bevorzugt erfolgt sie bei einer Temperatur bis 10°C. Sofern ein Niederschlag auftritt, wird dieser abfiltriert. Die erhaltene Lösung wird dann mit einem organischen, mit Wasser nicht mischbaren Lösungsmittel extrahiert. Beispiele für organische, mit Wasser nicht mischbare Lösungsmittel sind Ether, wie Diethylether, Dioxan, Kohlenwasserstoffe, wie Benzol, chlorierte Kohlenwasserstoffe, Methylenchlorid oder Chloroform oder deren Gemische. Nach dem Abtrennen der wäßrigen Phase wird diese dialysiert oder membranfiltriert, und das relaxinhaltige, aufbereitete Harnprodukt wird bei dem obigen Verfahren weiterverwendet.

Gemäß der ersten Stufe des erfindungsgemäßen Verfahrens wird die wäßrige, das Relaxin enthaltende Lösung auf eine Temperatur im Bereich über der Gefriertemperatur bis zu einer Temperatur von 15°C, vorzugsweise auf eine Temperatur im Bereich von 0-10°C und besonders bevorzugt auf eine Temperatur im Bereich von +2°C bis +6°C, gekühlt.

Zu der gekühlten Flüssigkeit gibt man dann eine starke Säure bis zu einem pH-Wert von 0,1 bis 2,5, vorzugsweise von 0,1 bis 1,0 und besonders bevorzugt von 0,2 bis 0,6. Die Ansäuerung erfolgt im allgemeinen unter Rühren im Verlauf von 5 Minuten bis 30 Minuten. Als konzentrierte Säure kann man alle konzentrierten Säuren verwenden, die die vorhandenen Enzyme nicht inaktivieren. Bevorzugt werden konzentrierte Salzsäure oder 20 bis 40 Gew.-%ige Schwefelsäure, Perchlorsäure, Trifluoressigsäure oder Trichlorsäure verwendet. Die Säurekonzentration kann variieren
und richtet sich nach dem gewünschten pH-Wert. Bevorzugt
verwendet man eine solche Konzentration, durch die eine
unnötige Verdünnung des Reaktionsgemisches vermieden wird.

Bei der Stufe zwei des erfindungsgemäßen Verfahrens wird
das Reaktionsgemisch während einer Zeit von 30 Minuten
bis 24 Stunden, vorzugsweise 3 bis 24 Stunden, besonders
bevorzugt 5 bis 15 Stunden, bei einer Temperatur im Bereich von der Gefriertemperatur bis 15°C, vorzugsweise
0-10°C, stehengelassen. Der Rückstand wird dann bei der
angegebenen Temperatur, beispielsweise durch Abfiltrieren
oder Abzentrifugieren, abgetrennt.

Zu der erhaltenen gekühlten Flüssigkeit gibt man dann
50 bis 200, vorzugsweise 60 bis 150 Vol.-%, bezogen auf
das Volumen der Flüssigkeit, an einem in Wasser löslichen
organischen Lösungsmittel zur Ausfällung der Proteine
hinzu. Die Menge kann vom Fachmann leicht bestimmt werden.
Die Zugabe des Lösungsmittels kann kontinuierlich oder
diskontinuierlich erfolgen. Unter in Wasser löslichen
Lösungsmitteln versteht man Lösungsmittel, die mindestens
zu 10% in Wasser löslich sind. Beispiele hierfür sind
Ketone, wie Aceton, Diethylketon, Methylethylketon ,
Alkohole, wie Methanol, Ethanol, Isopropanol und n-Propanol,
Ether, wie Diethylether und Dioxan, Acetonitril, Dimethylsulfoxid oder Dimethylformamid. Man kann auch Gemische
dieser Lösungsmittel verwenden.

Das erhaltene Reaktionsgemisch läßt man dann während einer
Zeit von 30 Minuten bis 24 Stunden, vorzugsweise 3 bis
24 Stunden und besonders bevorzugt 5 bis 15 Stunden, bei
der oben angegebenen Temperatur stehen. Der ausgefallene
Niederschlag wird dann in an sich bekannter Weise abgetrennt, wobei die Abtrennung beispielsweise durch Filtrieren, Zentrifugieren, Absetzen etc. erfolgen kann.

Zu der erhaltenen Flüssigkeit gibt man dann gemäß Stufe sieben des erfindungsgemäßen Verfahrens das 1- bis 10fache Volumen eines auf eine Temperatur von -10°C bis -70°C gekühlten, in Wasser löslichen organischen Lösungsmittels hinzu, wobei dieses Lösungsmittel das gleiche oder ein anderes wie bei der Stufe vier sein kann. Bevorzugt ist das Lösungsmittel auf eine Temperatur von -10°C bis -40°C gekühlt, und bevorzugt verwendet man die 2- bis 6fache Volumenmenge.

Bei dieser Stufe fällt ein Niederschlag aus, der gleich abgetrennt werden kann. Man kann das Reaktionsgemisch auch noch bei einer Temperatur vom Gefrierpunkt des Reaktionsgemisches bis +15°C stehen lassen, wobei das Stehenlassen während einer Zeit bis zu 24, vorzugsweise 3 bis 24, Stunden erfolgen kann. Dies ist immer dann sinnvoll, wenn die Ausfällung nicht vollständig ist. Es kann sich empfehlen, das Reaktionsgemisch während des Stehenlassens zu rühren. Dies ist jedoch nicht unbedingt erforderlich. Der erhaltene Niederschlag wird in an sich bekannter Weise von der Flüssigkeit abgetrennt, z.B. durch Zentrifugieren oder Filtrieren. Der Niederschlag enthält das Relaxin.

Aus dem Niederschlag wird dann das Relaxin in reiner Form gewonnen. Der Niederschlag wird gemäß Stufe zehn des erfindungsgemäßen Verfahrens in der 0,5- bis 5fachen, vorzugsweise 0,5- bis 2fachen, besonders bevorzugt 1fachen, Menge einer wäßrigen Pufferlösung, die einen pH-Wert im Bereich von 4,8 bis 5,6 aufweist, suspendiert. Bevorzugt verwendet man einen Ammoniumacetatpuffer mit einem pH-Wert von 5,3. Es können jedoch auch übliche Phosphatpuffer, die pH-Werte innerhalb der angegebenen Bereiche, vorzugsweise von 4,6 bis 6,2, aufweisen, verwendet werden.

Das erhaltene Reaktionsgemisch wird gegebenenfalls filtriert und gegebenenfalls mit 0,1 bis 0,5% Natriumazid und 0,05 bis 5 mM Phenylmethansulfonylfluorid oder -chlorid versetzt.

Das Relaxin ist jetzt in der Flüssigkeit enthalten. Das Reaktionsgemisch wird gegebenenfalls über ein Ultramembranfilter, welches eine Ausschlußgrenze von 10 000 bis 100 000 Dalton aufweist, vorzugsweise eine Ausschlußgrenze von 50 000 Dalton, filtriert. Beispiele für solche Filter sind die Amiconfilter oder die von der Firma Berghof gelieferten Filter.

Die erhaltene Flüssigkeit kann an dieser Stelle ebenfalls gegebenenfalls eingeengt werden. Gemäß den Stufen 12, 13 und 14 wird das Reaktionsgemisch oder eine das Relaxin enthaltende Fraktion über ein Ultramembranfilter filtriert oder/und einer Ionenaustauschchromatographie und/oder einer Gelfiltration unterworfen. Diese Stufen können beliebig oft durchgeführt werden, wobei es nicht immer erforderlich ist, alle Stufen durchzuführen. Die Gelfiltration ist jedoch zwingend.

Die Chromatographie von relaxinhaltigen Rückständen ist an sich bekannt, und die bekannten Verfahren können verwendet werden. Man kann beispielsweise die Flüssigkeit auf eine Carboxymethylcellulosesäule aufbringen. Die Säule kann vorher mit einem Puffer equilibriert worden sein. Nach der Auftragzeit spült man noch mit Puffer und eluiert dann das Protein mit einem linearen Salzgradienten. Die Reinigung erfolgt, wie in den oben angegebenen Literaturstellen beschrieben. Man erhält jetzt eine Probe, in der das Relaxin in konzentrierter Form zusammen mit anderen Proteinen vorliegt. Diese Probe kann erneut in Pufferlösung, wie oben angegeben, gelöst, chromatographiert und lyophilisiert werden. Die Reinigung erfolgt dann beispielsweise an einer Sephadex-G-Säule, wobei mit Ammoniumacetatpuffer eluiert wird. Man erhält nach mehrmaligen chromatographischen Reinigungen eine relaxinhaltige Probe, die für die verschiedenen Tests verwendet wird und in der das Relaxin in hoher Konzentration vorliegt.

Die Gelfiltration kann auch als Hochdruckgelfiltration durchgeführt werden. Als Gele kann man Sephadex, P-Gele von der Firma Bio Rad oder vergleichbare Gele (beispielsweise P6-Gel) TSK-Gele von Merck, etc. verwenden. Bei der Ionenaustauschchromatographie kann man, wie oben ausgeführt, Carboxymethylcellulose mit Divinylbenzol vernetzte Styrolsulfonsäureharze etc. verwenden.

In dem folgenden Schema ist die Gewinnung von Relaxin aus frischer Plazenta schematisch dargestellt:

Reaktionsschema für die Gewinnung
von Relaxin aus frischer Plazenta

Eine frische Plazenta, homogenisiert in 220 ml eiskaltem Ampuva

+    0,02% $NaN_3$

     2 mH Phenylmethansulfonylfluorid
+    32 ml konz. HCl
24 h, 4°C
+    170 ml Aceton
24 h, 4°C

     Zentrifugieren bei 4 000
     Upm während 30 min.

Überstand in 5facher Menge Aceton
(-70°C)

24 h Ausfällung

rohe Relaxinfraktion über $CaCl_2$ im Vakuum getrocknet

Suspendieren in 100 ml 50 mM $NH_4Ac$, pH 5,3 0,02% $NaNH_3$

geklärt durch Zentrifugierung bei 4 000 Upm während 30 min

Suspendieren in 5 ml 0,2 M $NH_4Ac$, pH 6,8, 0,02% $NaNH_3$ Klären durch Zentrifugieren bei 4 000 Upm während 30 min (Überstand filtriert)

Gelfiltration oder Sephadex-G50 (2,6 x 100 cm), Eluierung 0,2 M $NH_4Ac$, pH 6,8, 0,02% $NaN_3$ Strömungsrate 15 ml/h

Ionenaustauschchromatographie oder CM 52 (2,6 x 40 cm) Eluierung linearer NaCl Gradient: 0,2M - 0,5M Strömungsrate: 30 ml/h Hauptrelaxinfraktion, lyophilisiert, entsalzt an Sephadex-G10, lyophilisiert, suspendiert in 5 ml 0,2 m $NH_4Ac$, 6,8, 0,02% $NaNH_3$

Gelfiltration an Sephadex-G50 (2,6 x 100 cm) Eluierung: 0,2 M $NH_4Ac$, pH 6,8, 0,02% $NaN_3$

Strömungsrate: 18 ml/h

Fraktion aus biologisch aktivem Relaxin, lyophilisiert, entsalzt an Sephadex-G10, lyophilisiert, suspendiert in Ammoniumacetat

auf Sephadex-Ionenaustauschchromatographiesystem

Überraschenderweise wurde jetzt gefunden, daß das Relaxin, welches in der Vergangenheit als "rein" gegolten hat, noch in verschiedene Komponenten aufgetrennt werden kann. Die Anzahl der Komponenten hängt von dem verwendeten Ausgangsmaterial ab. Gegenstand der Erfindung ist somit, wie oben erwähnt, ebenfalls ein Verfahren zur Hochreinigung von Relaxin, das dadurch gekennzeichnet ist, daß man das Relaxin einer Hochreinigungsstufe unterwirft, indem man eine Umkehrphasenchromatographie, eine HPLC-Ionenaustauschchromatographie, eine HPLC-Gelchromatographie, ein Batch-Verfahren mittels Ionenaustauschchromatographie mit anschließender Elution und gegebenenfalls eine Gelchromatographie durchführt und die das Relaxin enthaltenden Fraktionen einengt. Die oben genannten Stufen für die Hochreinigung können beliebig oft wiederholt und in beliebiger Reihenfolge durchgeführt werden. Es ist auch nicht erforderlich, daß sämtliche Stufen durchgeführt werden.

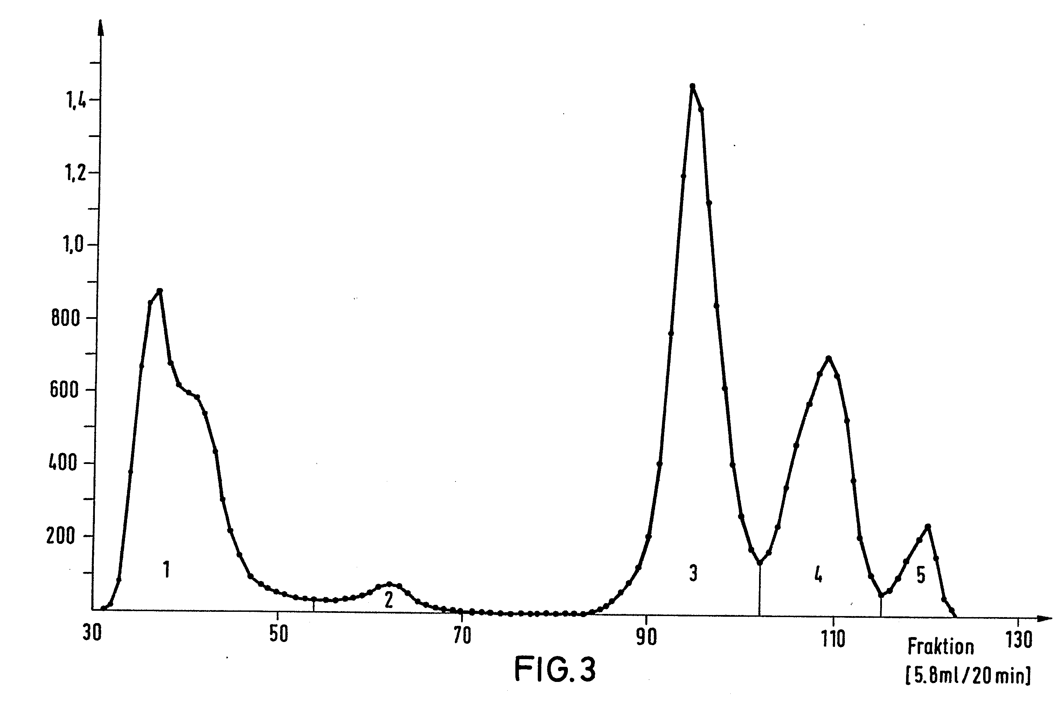

Bei der Hochdruck- oder Mitteldruckchromatographie verwendet man bevorzugt Ionenaustauschharze. Verwendet man bei der Hochdruck- oder Mitteldruckchromatographie Relaxin, welches in der Vergangenheit als reines Relaxin gegolten hat, so erhält man zwei Peaks, von denen nur einer pharmakologisch wirksam ist. Verwendet man dagegen bei der Hochdruck- oder Mitteldruckchromatographie Relaxin, welches aus Humanplazenta stammt und aus einer Carboxymethylcellulosesäule abgetrennt wurde, erhält man, wie in der beigefügten Fig. 3 erkennbar ist, vier Peaks, von denen ein Peak pharmakologisch wirksam ist.

Die Hochreinigung des Relaxins erfolgt mit einem präparativen Hochdruck- oder Mitteldruckchromatographiesystem, das speziell für die Ionenaustauschchromatographie von Peptiden und Proteinen an einem Kationenaustauscher ausgelegt ist (W. Voelter, H. Bauer, S. Fuchs und E. Pietrzik: J. Chromatogr. 153, 433-442 (1978); vgl. auch W. Voelter "High Performance Liquid Chromatography in Peptide Research" in der Monographie: High Performance Liquid Chromatography in Protein and Peptide Chemistry, Walter de Gruyter, Berlin, New York 1981). Auf die Offenbarung in dieser Literaturstelle wird expressis verbis Bezug genommen.

Für die Elution des Relaxins wird z.B. ein fünfstufiger Konzentrations- und pH-Gradient von Pyridin-Acetat-Puffer verwendet. Er überstreicht einen Konzentrationsbereich von 0,05 bis 3M Pyridin und einen pH-Bereich von pH 3,0 bis pH6. Zur Detektion werden ca. 4% des Eluats einer kontinuierlichen Partialhydrolyse in 6N Natronlauge unterworfen und entstandene freie Aminogruppen mit einem angesäuerten Ninhydrinreagens bei 570 nm photometrisch detektiert. Dadurch lassen sich auch Peptide ohne freie Aminogruppen und größere Peptide mit höherer Empfindlichkeit nachweisen. Die Nachweisgrenze liegt unter 1 µg je Fraktion.

Durch Lyophilisieren werden die isolierten Fraktionen als salzfreie Produkte direkt gewonnen, da Pyridin-Acetat-Puffer rückstandsfrei verdampfbar sind.

Bei der Trennung des Relaxins an einem stark sauren Ionenaustauscher auf der Basis eines quervernetzten Polystyroldivinylbenzolgerüsts werden mehrere Fraktionen erhalten. Die mittels des Mitteldrucksystems gewonnenen Fraktionen werden im Vakuum eingeengt, in bidest. Wasser aufgenommen und lyophilisiert.

In den beigefügten Figuren sind die erhaltenen Chromatogramme, die als Beispiele dienen sollen, dargestellt. Es zeigen:

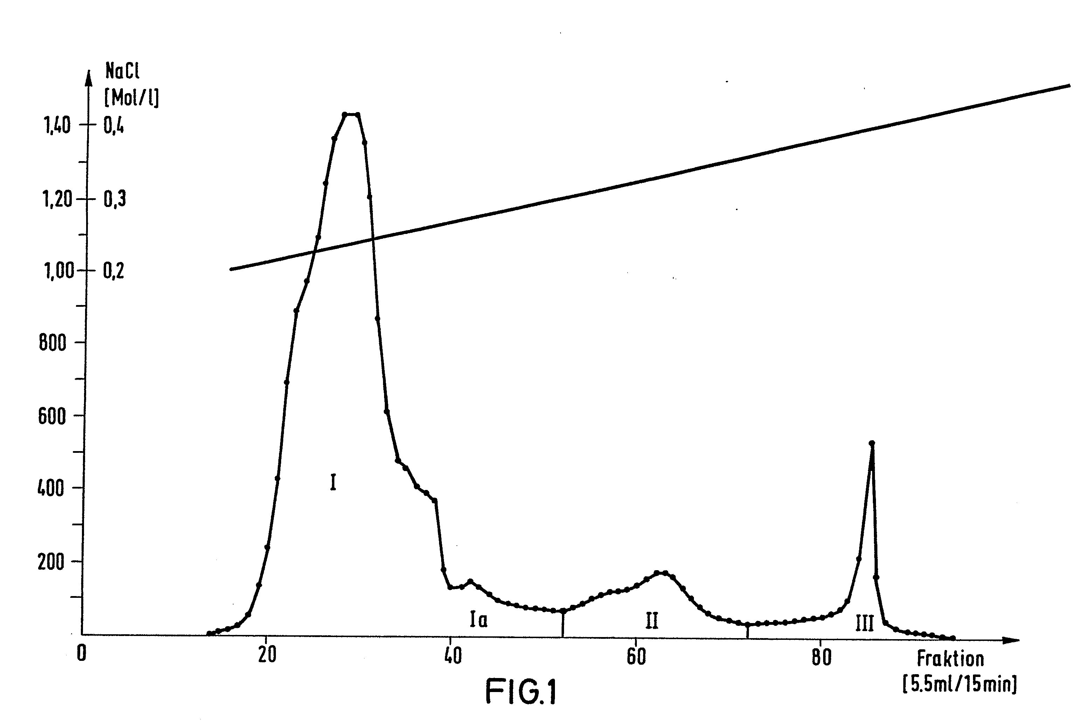

Figur 1 Kationenaustauschchromatogramm der rohen Relaxinfraktion aus humaner Plazenta, getrennt auf einer Carboxymethylcellulosesäule (2,6 x 40 cm). Equilibriert wurde mit 50 mM Ammoniumacetat (pH 5,3, 0,02% NaN$_3$). Die Proteinfraktionen wurden mit einem linearen NaCl-Gradienten von 0,2 M bis 0,5 M in 50 mM Ammoniumacetat (pH 5,3, 0,02% NaN$_3$) und einer Fließrate von 22 ml/h eluiert. Fraktionen wurden alle 15 min gesammelt. Detektiert wurde bei 280 nm.

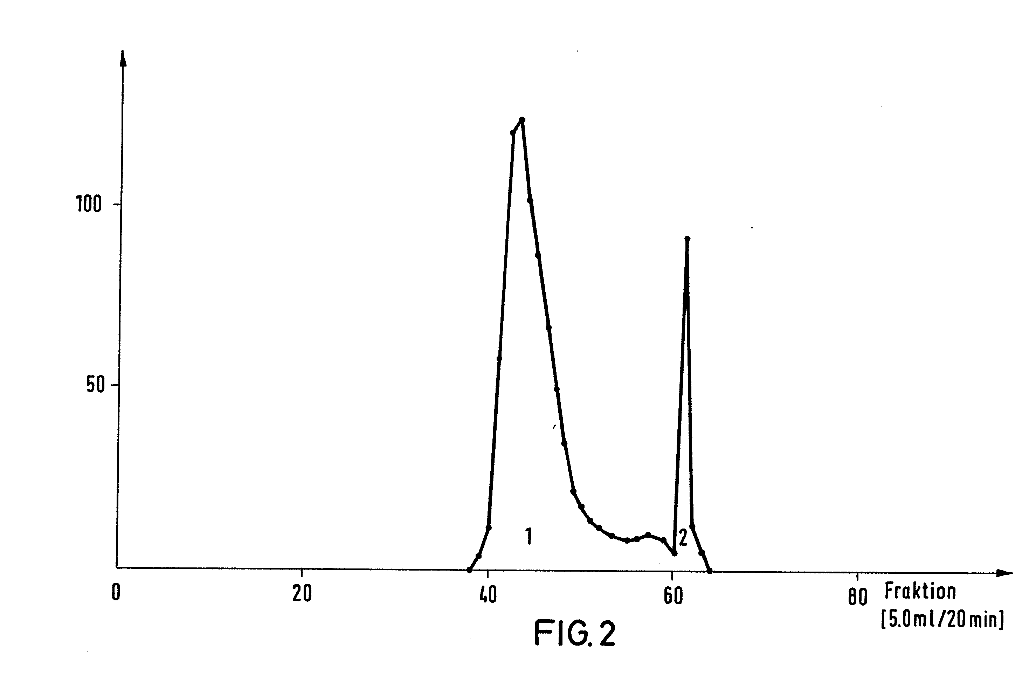

Figur 2 Gelfiltration der Hauptrelaxinfraktion III von Figur 1 auf einer Sephadex-G-50-Säule (2,6 x 100 cm). Equilibriert und eluiert wurde mit 0,2M Ammoniumacetat (pH 6,8, 0,02% NaN$_3$). Die Fließgeschwindigkeit war 15 ml/h. Fraktionen wurden alle 15 min gesammelt, und detektiert wurde bei 280 nm.

Figur 3 Gelfiltration der Rohrelaxinfraktion, die aus menschlicher Plazenta mit Hilfe einer Sephadex-G-50-Säule (2,6 x 100 cm) gewonnen wurde. Equilibriert und eluiert wurde mit 0,2M Ammoniumacetat (pH 6,8, 0,02% NaN$_3$). Die Fließrate war 15 ml/h. Fraktionen wurden alle 15 min gesammelt, und detektiert wurde wiederum bei 280 nm.

Zum Nachweis des Relaxins werden die bekannten Testverfahren, wie Radioimmunoassay oder Biotest, verwendet. Die Durchführung des Biotests erfolgt gemäß dem Verfahren von Steinitz et al (Endocrinol. 67, 102, 1966), die des RIA gem. E. Loumaye, B. Teuwissen und K. Thomas, Gynecol. Obstet. Invest., 9, 262-267,1978 .

Untersuchungen haben gezeigt, daß das Relaxin therapeutisch bei verschiedenen pathologischen Zuständen in der Schwangerschaft, wie vorzeitige Niederkunft und während der Geburt bei verzögerter Muttermundseröffnung, verwendet werden kann. Das Relaxin, insbesondere das Humanrelaxin, ist von äußerster Wichtigkeit für die Herstellung eines homologen Radioimmunoassaybestecks, das die Messung von Relaxin in menschlichen Körperflüssigkeiten und Geweben gestattet.

Überraschenderweise wurde jetzt auch gefunden, daß das Relaxin die Magensaftsekretion hemmt und eine Übersäuerung des Magens vermieden wird.

Die Erfindung betrifft insbesondere ein Mittel zur Behandlung von Störungen des Magen- und Darmtrakts, insbesondere bei der Behandlung von Magengeschwüren. Es war überraschend und hat nicht nahegelegen, daß Relaxin ein überraschend gutes Magen- und Darmmittel ist.

Die Erfindung betrifft weiterhin das bei dem obigen Verfahren erhaltene Relaxin.

Beispiel 1

Isolierung von Relaxin aus menschlicher Plazenta

Die Plazenta wird unmittelbar nach der Geburt in 220 ml eiskalter Ampuwalösung, welche 0,02% $NaN_3$ und 2 mmol Phenylmethansulfonylchlorid enthält, zunächst in einem Braun-Mixer und 3-4 mal 30 sek. in einem Ultrathorax (Janke und Kunkel, 220 V, 50 Hz, 10 000 U/min) homogenisiert.Nachdem man 32 ml eiskalte , konzentrierte Salzsäure zugefügt hat, wird der Brei über Nacht bei 4°C gehalten. Dann werden 170 ml Aceton zugefügt. Man läßt 24 h bei 4°C stehen und zentrifugiert schließlich bei 4 000 U/min 30 min lang. Zu der resultierenden Lösung werden 5 Vol. kaltes Aceton (-70°C) hinzugefügt. Das rohe Relaxin wird 24 h lang ausgefällt, durch Zentrifugation bei 4 000 U/min (20 min) lang gesammelt und über Calciumchlorid im Vakuum getrocknet.

Zur Weiterreinigung des rohen Relaxins wird dieses in 100 ml 50 mM Ammoniumacetatlösung (pH 5,3, 0,02% Natriumacid) suspendiert, durch Zentrifugation bei 4 000 U/min 30 min lang zentrifugiert und die überstehende Lösung auf eine Carboxymethyl-Cellulosesäule (2,6 x 40 cm) gebracht, mit 50 mM Ammoniumacetat equilibriert (pH 5,3, 0,02% Natriumacid). Das Protein wird mit einem linearen NaCl-Gradienten, der einen Bereich von 0,2M bis 0,5M hat, in derselben Pufferlösung mit einer Fließgeschwindigkeit von 30 ml/h eluiert. Die Fraktionen werden alle 15 min gesammelt und bei 280 nm mit einem Beckman-Photometer detektiert. Die relaxinhaltigen Fraktionen wurden gesammelt, durch Gelfiltration entsalzt (Sephadex-G-10) und wieder lyophilisiert. Die Hauptfraktionen wurden weiterhin durch Gelfiltration auf einer Sephadex-G-40-Säule (2,6 x 100 cm) equilibriert und zwar mit 0,2M Ammoniumacetat von pH 6,8, welches 0,02% Natriumacid enthielt. Das Protein wurde mit demselben Puffer mit einer Fließgeschwindigkeit von 18 ml/h eluiert. Die relaxinhaltige Fraktion wurde entsalzt und noch einmal lyophilisiert.

Zur Bestimmung des Relaxingehalts verschiedener Plazentas wurde der rohe Relaxinextrakt von einer Plazenta in 5 ml 0,2M Ammoniumacetat gelöst (pH 6,8, 0,02% NaN$_3$), durch Zentrifugation bei 4 000 U/min gereinigt (30 min lang) und direkt auf eine Sephadex-G-50-Säule aufgetragen, ohne vorher Ionenaustauschchromatographie anzuwenden. Die Fläche des Peaks bei 6 000 Dalton entspricht dem Relaxingehalt dieser Plazenta.

Beispiel 2

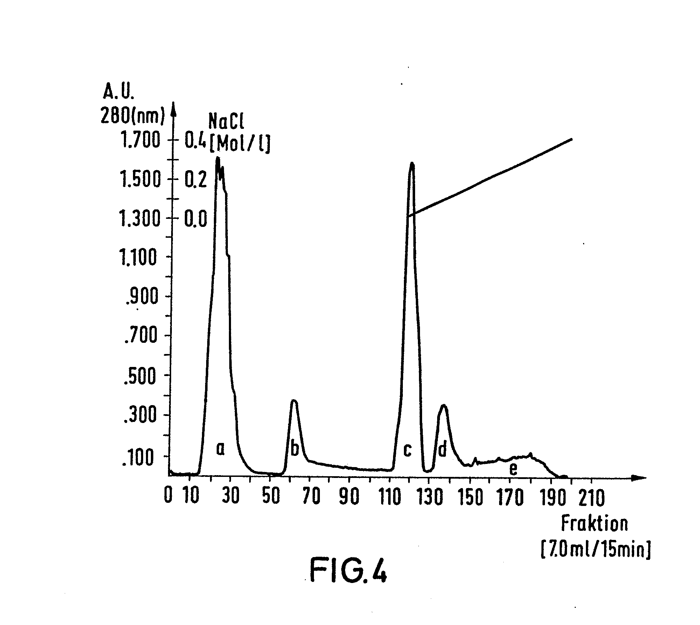

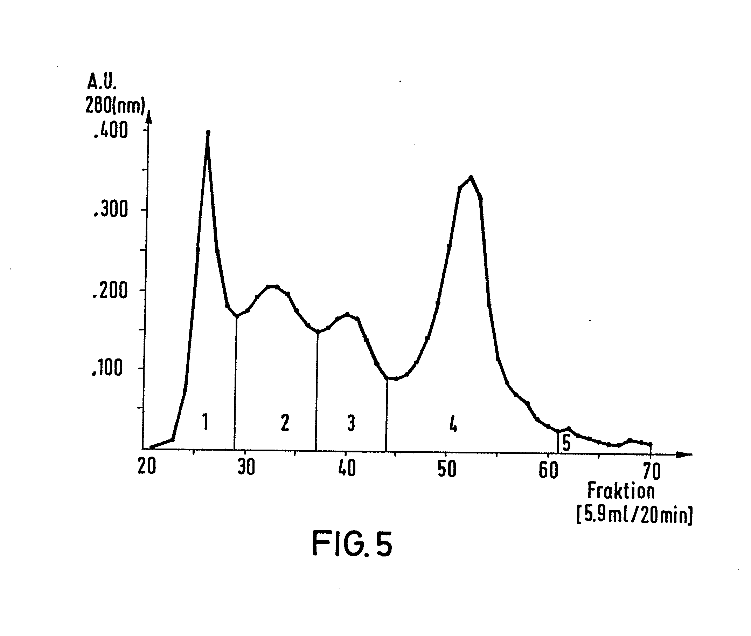

450 ml frische Samenflüssigkeit, die von der Hautklinik Tübingen erhalten wurde, wurde bei -70°C gelagert. Nach dem Auftauen wurden 0,03% Natriumazid und 2mM Phenylmethansulfonylchlorid zugegeben. Die Spermatozoa werden von Samenplasma durch Zentrifugieren bei 4 000 Upm (20 min.) abgetrennt. Das rohe Relaxin wird durch saure Acetonextraktion bei 4°C entsprechend dem Verfahren von Doczi isoliert. Der erhaltene Niederschlag wird in 80 ml 50 mM Ammoniumacetatpuffer (pH 5,3, 0,02% NaN$_3$) suspendiert und bei 4 500 Upm (30 min.) zentrifugiert. Nach der Filtration erhält man eine klare, gelbe Flüssigkeit, welche bei 4°C gelagert wird. Die Reinigung erfolgt durch Ionenaustauschchromatographie an einer Carboxymethylcellulose-52-Säule, die mit 50 mM Ammoniumacetatpuffer (pH 5,3, 0,02% NaN$_3$) equilibriert ist. Nach der Eluierung des nicht-absorbierten Proteins mittels eines gepufferten Lösungsmittels wird das absorbierte Protein mit einem linearen Natriumchloridgradienten (0,0 - 0,4 M) ausgesalzt. Nach dem Entsalzen an einer Sephadex-G10-Säule wird die Relaxin enthaltende Fraktion getrennt in eine equilibrierte Sephadex-G50-Säule übertragen und dann wird mit 0,2 M Ammoniumacetatpuffer (pH 6,8, 0,02% NaN$_3$) eluiert (Elutionsgeschwindigkeit 18 ml/h). Die Fraktionen werden alle 20 min. gesammelt und bei 280 nm nachgewiesen. Nach dem Entsalzen, wie oben beschrieben, wird der Relaxinpeak mit der höchsten biologischen Aktivität (entsprechend dem mäuseinterpubischen Ligamentbioassay von Steinetz) an einer Flüssigkeitschromatographievorrichtung mit hoher Leistung getrennt. Das Relaxin wird an Carboxymethylcellulose gereinigt. In der beigefügten Fig. 4 sind die erhaltenen Peaks dargestellt. Das Relaxin wurde von den anderen Proteinen (a,b) abgetrennt und in zwei Fraktionen (c,d) mit 0,04 M und 0,12 M Natriumchloridkonzentration eluiert. Die Fraktionen, die dem Hauptrelaxinpeak (c) entsprechen, werden gesammelt, lyphilisiert, entsalzt und erneut lyophilisiert und durch Gelfiltration an einer Sephadex-G50-Säule (Fig. 5) gereinigt. Wie aus Fig. 5 hervorgeht, enthält diese Relaxinfraktion mindestens vier Proteine mit unterschiedlichem Molekulargewicht.

Fig. 4: Kationenaustauschchromatogramm der rohen Relaxinfraktion, die aus Samenfluid extrahiert wurde, Carboxymethyl-Cellulosesäule (2,6 x 40 cm), equilibriert mit 50 mM Ammoniumacetat (pH 5,3, 0,02% $NaN_3$). Die relaxinfreien Proteine werden mit 50 mM Ammoniumacetatpuffer eluiert. Die relaxinenthaltenden Proteinfraktionen werden mit einem linearen NaCl-Gradienten von 0,0 bis 0,4 M in 50 mM Ammoniumacetat, Strömungsrate 28 ml/h, eluiert. Die Fraktionen werden alle 15 min. gesammelt und bei 280 nm nachgewiesen.

Die biologisch aktive Fraktion 4 wurde weiter an einem Hochleistungsionenaustauschchromatographiesystem analysiert. Das Eluierungsmuster zeigt drei Hauptfraktionen des Relaxins.

Fig.5: Gelfiltrationschromatogramm (Sephadex-G50, 2,6 x 100 cm, equilibriert, Eluierungsmittel 0,2 M Ammoniumacetat, pH 6,8, 0,02% $NaN_3$, 24 ml/h) der Fraktionen, die den Peak c in Fig. 4 darstellen.

Gelfiltration des Relaxinpeaks d von der ersten Reinigungsstufe (Fig. 4) ergibt nur einen einfachen Peak bei dem gleichen Eluierungsvolumen wie der Peak 4 in Fig. 4.

Dies bedeutet, daß das Molekulargewicht der beiden Relaxinfraktionen, die sich in ihrer Ladung unterscheiden, ungefähr identisch ist. Es existieren mindestens zwei Relaxine mit unterschiedlicher Ladung, aber identischem Molekulargewicht.

Nachweis des Relaxins:

Der Nachweis des Relaxins erfolgte nach dem Radioimmunoassay (RIA) oder nach dem Biotest (vgl. die oben genannten Literaturstellen).

Durchführung des Radioimmunoassays:

Zur Jodierung werden 5µg Relaxin, gelöst in 10 µl 0,1 m Boratpuffer (pH = 8,5) zu 4 mCi Bolton-Hunter-Reagenz gegeben und 15 min. lang bei 0°C geschüttelt. Dazu gibt man 0,5 ml einer 0,2 m Glycinlösung in 0,1 m Boratpuffer und schüttelt wieder bei 0°C 5 min. lang. Die Trennung des Relaxins erfolgt durch Gelchromatographie mit einer Sephadex-G-25-Säule (25 x 1 cm) mit 0,05 m Phosphatpuffer (pH = 7,5). Die das radioaktiv markierte Relaxin enthaltenden Fraktionen werden mit 0,5 ml einer Pufferlösung PM 16 mit 5% Ovalbumin verdünnt und als Stammlösung für den Assay benutzt. Für die Standardkurve werden jeweils 100 µl der verschiedenen Standardlösungen (156 - 20 000 pg Relaxin pro ml PM 16) sowie 300 µl eines SMA-Kontrollserums und 100 µl PM 16 Pufferlösung zugegeben. Dazu gibt man Antikörperverdünnung, die wie folgt gewonnen wird.

Antiserum R6 wird im Verhältnis 1 / 36 000 mit EDTA-Puffer (0,05 m EDTA - $Na_2$ in PM 16-Puffer mit NaOH auf 7,2 eingestellt und mit 1/60 Kaninchenserum versetzt) verdünnt.Man läßt 1 h bei +4°C stehen und fügt dann 100 11 Tracerlösung (Stammlösung des jodierten Relaxins durch Verdünnen mit PM 16/1% Ovalbumin auf ca. 10 000 cpm/100 ml gebracht) zu. Man inkubiert 24 h bei +4°C. Die Fällung des Antigen-Anti-

**0107782**

körper-Komplexes erfolgt nach der Doppelantikörpermethode mit 200 µl Kaninchenglobulin-Antikörper 1:4 mit PM 16/1% 200 µl Ovalbumin verdünnt. Nach 24 h bei +4°C wird bei 4 000 g zentrifugiert und nach Abdekantieren im Gammezähler gemessen.

Die unspezifische Bindung ist ein Maß für die Wechselwirkung zwischen Antigen und unspezifischen Antikörpern und wird durch Zugabe von 100 µl des EDTA-Puffers zu 300 µl Kontrollserum bestimmt. Man verfährt nach Zugabe von 100µl Tracerlösung, wie oben beschrieben.

Die Nullbindung ist Ausdruck der Bindung zwischen Antigen und Antikörper und wird durch Zugabe von 200 µl PM 16/1% Ovalbumin, 100 µl Antiserumverdünnung und 100% Tracerlösung zu 300 µl Kontrollserum bestimmt.

Ende der Beschreibung.

Patentansprüche

1.    Verfahren zur Herstellung von Relaxin, dadurch g e - k e n n z e i c h n e t ,   daß man ein gegebenenfalls vorbehandeltes, relaxinhaltiges Ausgangsmaterial

(1)    mit einer starken Säure bis zu einem pH-Wert von 0,1 bis 2,5 bei einer Temperatur im Bereich von der Gefriertemperatur bis 15°C im Verlauf von 5 Minuten bis 30 Minuten ansäuert,

(2)    das Reaktionsgemisch während einer Zeit von 30 Minuten bis 24 Stunden bei einer Temperatur im Bereich von der Gefriertemperatur bis 15°C stehen läßt,

(3)    den Rückstand bei einer Temperatur im Bereich von der Gefriertemperatur bis 15°C abtrennt,

(4) zu der erhaltenen klaren Flüssigkeit bei einer Temperatur im Bereich von der Gefriertemperatur bis 15°C, 50 bis 200 Vol-% bezogen auf das Volumen der Flüssigkeit an einem in Wasser löslichen, organischen Lösungsmittel zugibt,

(5) die Stufe 2 wiederholt,

(6) die Stufe 3 wiederholt,

(7) zu der erhaltenen Flüssigkeit das 1- bis 10fache Volumen eines auf eine Temperatur von -10°C bis -70°C gekühlten, in Wasser löslichen, organischen Lösungsmittels zugibt, wobei dieses Lösungsmittel das gleiche oder ein anderes wie bei der Stufe 4 sein kann,

(8) die Stufe 2 gegebenenfalls wiederholt,

(9) den das Relaxin enthaltenden Niederschlag bei einer Temperatur im Bereich von der Gefriertemperatur bis 15°C abtrennt,

(10) den Niederschlag zu der 0,5- bis 5fachen Volumenmenge einer Pufferlösung zugibt und gegebenenfalls filtriert,

(11) gegebenenfalls 0,1 bis 0,5% Natriumazid und 0,05 bis 5 mM Phenylmethansulfonylfluorid oder -chlorid zu dem erhaltenen Reaktionsgemisch zugibt,

(12) das Reaktionsgemisch bzw. die das Relaxin enthaltenden Fraktionen über ein Ultramembranfilter filtriert, welches eine Ausschlußgrenze von 50 000 Dalton aufweist und/oder,

(13) das Reaktionsgemisch bzw. die das Relaxin enthaltenden Fraktionen einer Ionenaustauschchromatographie unterwirft,

(14) das Reaktionsgemisch bzw. die das Relaxin enthaltenden Fraktionen einer Gelfiltration unterwirft,

wobei die Stufen 12, 13 und 14 in beliebiger Reihenfolge und mehrmals durchgeführt werden können und wobei die Stufe 14 zwingend ist und mindestens eine der Stufen 12 oder 13 durchgeführt werden muß und,

(15) die das Relaxin enthaltenden Fraktionen einengt und das gereinigte Relaxin gewinnt.

2. Verfahren nach Anspruch 1, dadurch g e k e n n z e i c h n e t , daß man zur Hochreinigung von Relaxin eine Lösung von gereinigtem Relaxin einer Hochreinigungsstufe unterwirft, indem man eine

(16) Umkehrphasenchromatographie oder/und

(17) eine HPLC-Ionenaustauschchromatographie oder/und

(18) HPLC-Gelchromatographie oder/und

(19) ein Batch-Verfahren mittels Ionenaustauschchromatographie mit anschließender Elution und

(20) gegebenenfalls eine Gelchromatographie durchführt und

(21) die das Relaxin enthaltende Fraktion einengt und das hochgereinigte Relaxin gewinnt,
wobei die Stufen 16 bis 19 gegebenenfalls beliebig
wiederholt werden können und wobei mindestens zwei
der Stufen 16 - 19 durchgeführt werden müssen.

3. Verfahren zur Herstellung von Relaxin nach Anspruch
1, dadurch g e k e n n z e i c h n e t , daß man als
relaxinhaltiges Ausgangsmaterial Spermienflüssigkeit,
Mucosa, Teile und Ausscheidungsprodukte des Magen- und
Darmtrakts, Ovarien oder Corpus luteum verwendet.

4. Verfahren zur Herstellung von Relaxin nach Anspruch
1, 2 oder 3, dadurch g e k e n n z e i c h n e t , daß
man zur Herstellung einer vorbehandelten relaxinhaltigen
Spermienflüssigkeit Samenejakulat mit 0,01 bis 0,1%
Natriumazid, gegebenenfalls mit 0,05 bis 5 mM Phenylmethansulfonylfluorid oder -chlorid, versetzt und in Spermatozoa
und Spermienflüssigkeit trennt und die erhaltene Spermienflüssigkeit weiterverwendet.

5. Verfahren zur Herstellung von Relaxin nach Anspruch
1, 2 oder 3, dadurch g e k e n n z e i c h n e t , daß
man zur Herstellung einer vorbehandelten relaxinhaltigen
Ovarienmischung frische Plazenta in eiskalter Ampuva-
Lösung, die 0,01 bis 0,1% Natriumazid und 0,05 bis 5 mM
Phenylmethansulfonylfluorid oder -chlorid enthält, homogenisiert.

6. Verfahren zur Herstellung von Relaxin nach Anspruch
1, 2 oder 3, dadurch g e k e n n z e i c h n e t , daß
man zur Herstellung eines vorbehandelten relaxinhaltigen
Harns

(1) Harn auf einen pH-Wert im Bereich von 0,1 bis 5
ansäuert,

(2)     die unlöslichen Materialien abtrennt,

(3)     gegebenenfalls bei einer Temperatur bis 40°C
        einengt und, sofern ein Niederschlag auftritt,
        diesen abfiltriert,

(4)     die erhaltene Lösung mit einem organischen, mit
        Wasser nicht mischbaren Lösungsmittel extrahiert,

(5)     die wäßrige Phase abtrennt,

(6)     die wäßrige Phase dialysiert oder membran-
        filtriert und das relaxinhaltige, aufbereitete
        Harnprodukt weiterverwendet.

7.    Verfahren zur Herstellung von Relaxin nach Anspruch
1, dadurch g e k e n n z e i c h n e t , daß man als
starke Säure konzentrierte Salzsäure, 20 bis 40 Gew.-%ige
Schwefelsäure, Perchlorsäure, Trifluoressigsäure oder Trichloressigsäure verwendet.

8.    Verfahren zur Herstellung von Relaxin nach Anspruch
1, dadurch g e k e n n z e i c h n e t , daß man als
organisches wasserlösliches Lösungsmittel Ketone wie
Dialkylketone, wie Aceton; Alkohole, Ether wie Dioxan,
Acetonitril, Dimethylsulfoxyd oder Dimethylformamid oder
Gemische dieser Verbindungen verwendet.

9.    Verfahren zur Herstellung von Relaxin nach Anspruch
1, dadurch g e k e n n z e i c h n e t , daß man als
Pufferlösung Ammoniumacetatpuffer mit einem pH-Wert von
4,8 bis 5,6, vorzugsweise mit einem pH-Wert von 5,3 oder
Phosphatpuffer mit einem pH-Wert von 4,6 bis 6,2 verwendet.

10. Verfahren nach Anspruch 6, dadurch g e k e n n - z e i c h n e t , daß man als organisches mit Wasser nicht mischbares Lösungsmittel Ether, Kohlenwasserstoffe, chlorierte Kohlenwasserstoffe oder deren Gemische verwendet.

11. Verfahren nach Anspruch 6, dadurch g e k e n n - z e i c h n e t , daß man zum Ansäuern Salzsäure, Schwefelsäure, Perchlorsäure, Trifluoressigsäure oder Trichloressigsäure verwendet.

12. Relaxin dadurch g e k e n n z e i c h n e t , daß es nach einem der Verfahren der Ansprüche 1 bis 11 erhalten worden ist.

13. Arzneimittel dadurch g e k e n n z e i c h n e t , daß es neben üblichen Trägerstoffen und Verdünnungsmitteln Relaxin, welches aus Spermienflüssigkeit, Mucosa, Teilen und Ausscheidungsprodukten des Magen- und Darmtrakts, Ovarien oder Corpus luteum erhalten worden ist, enthält.

14. Arzneimittel dadurch g e k e n n z e i c h n e t , daß es neben üblichen Trägerstoffen und Verdünnungsmitteln Relaxin nach Anspruch 12 enthält.

FIG.1

FIG.2

FIG.3

0107782

FIG.4

FIG. 5

0107782

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | Chemical Abstracts Band 80, Nr. 13, 1. April 1974, Columbus, Ohio, USA C.D. SHERWOOD et al. "Purification and characterization of porcine relaxin", Seite 94, Spalte 1, Abstract Nr. 67606x & Arch. Biochem. Biophys., Band 160, Nr. 1, 1974, Seiten 185-196 (Kat. A, D) | 1,8 | C 07 G  15/00<br>C 07 C 103/52<br>A 23 J   1/00<br>A 61 K  35/00<br>A 61 K  37/24 |
| A | Chemical Abstracts Band 88, Nr. 11, 13. März 1978, Columbus, Ohio, USA M. WEISS et al. "Purification procedure for the peptide hormone relaxin", Seite 224, Spalte 1, Abstract Nr. 71076g & Arch. Gynaekol., Band 223, Nr. 3, 1977, Seiten 2 33-239 | 1 | |

RECHERCHIERTE
SACHGEBIETE (Int. Cl. ³)

| A | Chemical Abstracts Band 96, Nr. 17, 26. April 1982, Columbus, Ohio, USA M.J. FIELDS et al. "Octadecylsilica and carboxymethyl cellulose isolation of bovine and porcine relaxin", Seite 107, Spalte 2, Abstract Nr. 136066r & Ann. N.Y. Acad. Sci., Band 380, 1982, Seiten 36-46 (Kat. A, D) | 1 | A 23 J   1/00<br>A 61 K  35/00<br>A 61 K  37/24<br>C 07 C 103/52<br>C 07 G  15/00 |

--- -/-

Der vorliegende Recherchenbericht wurde für alle Patentansprüche erstellt.

| Recherchenort<br>BERLIN | Abschlußdatum der Recherche<br>21-12-1983 | Prüfer<br>KNAACK M |
|---|---|---|

**Europäisches Patentamt**

# EUROPÄISCHER RECHERCHENBERICHT

| | EINSCHLÄGIGE DOKUMENTE | | Seite 2 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | Chemical Abstracts Band 96, Nr. 25, 21. Juni 1982, Columbus, Ohio, USA S. YAMAMOTO et al. "The isolation of relaxin from boar testis", Seite 96, Spalte 2, Abstract Nr. 211088e & Relaxin, Proc. Workshop Chem. Biol. Relaxin, Band 1980, Seiten 71-74 | 1,9 | |

-----

**RECHERCHIERTE SACHGEBIETE (Int. Cl. ³)**

Der vorliegende Recherchenbericht wurde für alle Patentansprüche erstellt.

| Recherchenort | Abschlußdatum der Recherche | Prüfer |
|---|---|---|
| BERLIN | 21-12-1983 | KNAACK M |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

                          
& : Mitglied der gleichen Patentfamilie, übereinstimmendes Dokument

EPA Form 1503 03 82